# EUROPEAN PATENT APPLICATION

(11) **EP 3 081 550 A1**
(43) Date of publication of application: **19.10.2016**
(21) Application number: 13898898.5
(22) Date of filing: 11.12.2013
(51) Int. Cl.: C07C 69/34, C07C 69/704, C07C 69/003, A01N 25/00, A01N 25/22

(54) **POLYCARBOXILIC ACID ALKYL ESTER DERIVED FROM BRANCHED AND LINEAR ALCOHOL OF PLANT ORIGIN, AND USE OF AN ALKYL ESTER IN AGROCHEMICAL FORMULATIONS**

(71) Applicant: Oxiteno S.A. Industria e Comercio, 01317-910 Sao Paulo - SP (BR)
(72) Inventor: DE OLIVEIRA, Adriano, Sales, 09040-270 Santo André - SP (BR); DE SOUSA, Ubiratan, Ferreira, 70673-311 Brasília - DF (BR); SOUZA, Tiago, de Moraes e, 05687-001 São Paulo - SP (BR); AVILA, Oscar, Osvaldo, Hernandez, 44920 Guadalajara, Jalisco (MX)
(74) Representative: Eisenführ Speiser
(86) International application number: PCT/BR2013/000551
(87) International publication number: WO 2015/085377

(57) **Abstract**

The invention pertains to a polycarboxylic acid ester alkyl derived from branched and straight alcohol of a plant origin, comprising C4 and C5 chain alcohols or admixtures thereof adding up to more than 90% by weight of the total esters, and also to the use thereof in agrochemical formulations.

## Description

### Field of the Invention

The present invention pertains to solvents derived from a natural and renewable source in the form of polycarboxylic acid alkyl esters obtained from branched alcohols of plant origin, as an alternative to solvents derived from hydrocarbons used in the preparation of agrochemical formulations. This invention also describes agrochemical formulations comprising the above polycarboxylic acid alkyl esters as solvent.

### Background of the Invention

The most effective way of combating pests in crops is through the application of pesticides following appropriate management practices. According to the present invention, pesticides comprise all and any substance, ingredient or active ingredient (AI), or preparation, in neat or diluted form, which is applied on crops for the purpose of controlling or mitigating pests. Pesticides can be classified according to the pests they combat, and among the most common found on the market are herbicides, insecticides, fungicides and acaricides.

Usually pesticides are found on the market in both neat form and incorporated in agrochemical formulations that generally comprise one or more AIs and other substances classified as inert which boost their effects and facilitate their application, such as carriers, adjuvants or additives. Such formulations can be applied directly to crops or, more commonly, after dilution and formation of the so-called spray mixture. The type of formulation employed is defined primarily based on the physical-chemical characteristics of the AI(s), and can be: soluble concentrate (SL), emulsifiable concentrate (EC), emulsion in water (EW), suspension concentrate (SC), suspoemulsion (SE), microemulsion (ME), dispersion or suspension concentrate in oil (OD), dispersible concentrate (DC), encapsulated suspension (CS), dispersible granule (WG), wettable powder (WP), among others (ABNT NBR 12679:2004 - Agrotoxics and the like, Technical products and formulations, Terminology).

The various types of agrochemical formulations are the result of the existence of a large variety of AIs having different chemical natures. The easiest way to promote the delivery of AIs to their respective targets is through solubilization thereof in water, although some of these AIs are not soluble in water. Many AIs do not present the physical-chemical characteristics which are required for being stabilized in the form of solid micrometric particles suspended in water, to be prepared in the form of an SC. Accordingly, a shape widely used to solubilize AIs is made from organic solvents from different classes, leading to EC-type formulations. The solvents traditionally used for preparing EC-type agrochemical formulations are solvents of the class of hydrocarbons. Among those commonly used are aromatic solvents, which after several years of use and studies, have been recognized as harmful due to their severely toxic characteristics to humans and the environment.

The search for less toxic solvents for use in agrochemical formulations has been increasingly explored. Patent US 6,955,818 describes solvents derived from esters for obtaining formulations with active ingredients that are typically used as insecticides/acaricides for veterinary treatments with lesser irritability appeal. Another reference is patent US 7,776,792, which describes alkyl esters of polycarboxylic acids as solvents of AIs herbicides inhibitors of acetate lactate synthase (ALS). Another example is patent US 4,672,072 which refers to the use of organic solvents such as Xylene and Toluene, among others, as carrier of veterinary formulations but preferably references the use of these solvents in a combined form with vegetable oils which shows how desirable it is to obtain formulations that dissolve active ingredient pesticides with a lower share of aromatic solvents.

However, the use of vegetable oils as such, or even in the form of mixtures, has the restriction of presenting a high freezing point, which limits its use in formulations when exposed to low temperatures due to the freezing behavior thereof. In other cases, when obtaining alternative solvents having sufficiently low freezing points, it is common for these to exhibit high water solubility, which hampers the development of new EC-type formulations. Another disadvantage of some of these inventions is that they are limited to formulations of a specific class of AIs, such as in US 7,776,792.

It has now surprisingly been found that ester polycarboxylates, specifically derived from branched alcohols of plant origin, exhibit excellent solvency power for different classes of AIs, including insecticides, acaricides, fungicides and herbicides, allowing the solubilization of these AIs in agrochemical formulations with better toxicological profiles than the aromatic hydrocarbon solvents. Additionally, they exhibit low freezing points and low solubility in water, favoring the development of EC-type formulations.

Therefore, objects of the present invention are solvents derived from a natural and renewable source in the form of polycarboxylic acid alkyl esters obtained from branched alcohols of plant origin as an alternative to solvents derived from hydrocarbons used in the preparation of agrochemical formulations. Another object of the present invention are agrochemical formulations comprising the above polycarboxylic acid alkyl esters as solvent. The advantages of the agrochemical formulations of the present invention will become apparent in the description below.

### Detailed Description of the Invention

The polycarboxylic acid alkyl esters referred to in this invention are obtained by conventional sterification processes from branched alcohols of plant origin and polycarboxylic acids.

The branched alcohols of plant origin suitable for use in this invention are C4 and C5 chains and are subjected neat or in admixture among them to the sterification reaction with the polycarboxylic acids. These C4 and C5 chain alcohols described are obtained from distillation of fusel oil. The fusel oil is a by-product generated from the distillation process of ethyl alcohol, from the fermentation of various plant sources such as sugar cane, corn and cellulose.

In a preferred embodiment of this invention, the polycarboxylic acid ester alkyl is tri-isobutyl citrate, obtained by the reaction of isobutyl alcohol and citric acid. In another preferred embodiment, the solvent obtained by the reaction of the isopentyl alcohol and citric acid, leading to tri-isopentyl citrate.

Due to the chemical nature of the alkyl esters of polycarboxylic acids of this invention, these solvents individually or in admixtures are highly compatible with a wide variety of AIs. Additionally, these solvents can perform other functions in an agrochemical formulation as a co-solvent or anti-crystallizing agent. Other advantages of this invention is that the cited alkyl esters of polycarboxylic acids exhibit desirable properties for an EC-type agrochemical formulation as a freezing point below -20°C, flash point in a closed vessel over 115°C, classifying the same as non-flammable and insoluble in water.

The alkyl ester of polycarboxylic acids described in this invention are readily incorporated into agrochemical formulations of the SL, EC, EW, SC, SE, ME, OD, DC, CZ type, comprising one or more pesticides.

Based on the commonly used nomenclature (The Pesticide Manual, Tomlin, C. D. S. (Ed.), 2006, 14th edition, British Crop Production Council), examples of pesticides with which the solvents of this invention have compatibility include the following classes of herbicides: 4-(aryloxyphenoxy) alkanoic acid, 2-(4-aryloxyphenoxy) alkanoic acid, 1,3,5-triazine, 1,3,5-triazine-2,4-dione, 2-(aryloxyphenoxy) propionamide, 2,6-dinitroaniline, 2-chloroacetanilide, acetamide, anilide, aromatic acid, arylaminopropionic acid, aryloxycarboxylic acid, aryloxyphenoxy propionate, benzamide, benzene dicarboxylic acid, benzimidazole, benzofuran, benzoic acid, benzonitrile, benzothiadiazinone, bipyridilium, carbamate, chloroacetamide, cyclohexanodione oxime, dinitroaniline, dinitrophenol, diphenyl ether, glycine derivative, halogenated alkanoic acid, hydroxybenzonitrile, imidezolinone, isaoxazole, isoxazolidinone, N-phenylphthalimide, organoarsenic, organochloride, organophosphorus compounds, oxadiazole, oxyacetamide, phenoxy carboxylic acid, phenyl carbamate, phenylpyrazole, phenyl pyridazine, phenylurea, phosphinic acid, phosphoramidate, phosphorodithioate, phthalamate, pyrazole, pyridazine, pyidazinone, pyridin, pyridinecarboxamide, pyridinecarboxylic acid, pyrimidindione, pyrimidine, pyimidinyloxybenzoic acid, quinolinocarboxylic acid, thiocarbamate, semicarbazone, sulfonylurea, thiadiazole, thiocarbamate, triazine, triazinone, triazole, triazolinone, triazolocarboxamide, triazolopyrimidine, triketone, uracyl and urea. Examples of classes of fungicides include: qil, strobilurin-type, strobilurin analog, triazole, phenylamide, benzimidezole, vanylamide carbamate, aromatic hydrocarbon, pyidine carboxyamide, aminopyrimidinole, phthalimide, oxatin carboxyamide, cyclopropane carboxyamide, chlorophenylnitroaniline, chloronitrile, phenylacetamide, cyanoacetamide oxime, anilinopyrimidine, alkylenebis (dithiocarbamate), sulfamide, carboxyamide, pyridazinone, N-phenyl carbamate, pyrimidinamine, cinnamic acid amide, quinone, morpholine, guanidine, phosphorothiolate, thiazole carboxyamide, pyrimidine, furancarboxyamide, hydroxy anilide, propionamide, piperidine, organotin, dimetildithiocarbamate, 2,6-dinitroaniline, phenylpyrrol, benzamide, pyridinyl ethylbenzamide, benzenosulphonamide, phenyl benzamide, phosphonate, pyrazole carboxyamide, guanidine, imidazole, dicarboxyamide, mandelamide, benzophenone, butyrolactone, phenylurea, isobenzofuranone, piperidine, polyoxin, carbamate, quinazolinone, pyrroloquinolinone, quinoline, thiophenacarboxyamide, spiroketalamine, thiadiazolcarboxamide, benzotriazine, triazolobenzothiazole, piperazine, glucopyranosyl, 3'-4'-dichloroanilide, organomercury, triazine, urea, enupiranuronic acid, anilide, aminopyrimidinole, organophosphate, diazosulphonate, organoarsenic and pyridin.

The solvents are also compatible, in different concentrations, with different classes of insecticides, including organophosphate, neonicotinoid, oxime carbamate, pyrethroid, carbamate, nereistoxin analog, cyclodiene organochloride, bezoylurea, avermectin, phenylpyrazole, juvenile hormone mimic, diacylhydrazine, oxadiazine, selective food blocker, fumigant, dimethylcarbamate, phenoxy carboxylic acid precursor, organophosphorous compounds, carbamoyloxime and urea; in addition to various acaricides, such as, for example, those belonging to the organotin, benzylate, termite growth inhibitors, METI, pyrethroid, organochloride, benzymidazole and organophosphate classes.

It is important to mention that the solvents of this invention are also compatible with various inert ingredients and adjuvants commonly used in agrochemical formulations. Among the inert ingredients with which the solvents of this invention are compatible are various surfactants, which can be anionic, cationic, amphoteric and non-ionic. Said compatibility mean the solvents of this invention can be used without restrictions in terms of the type of agrochemical formulation and components thereof.

The composition of solvents of the invention comprises, among the various active ingredients cited, the preferred solubilization of the following AIs: Abamectin (Avarmectin), Bifenthrin (Pyrethroid), Cypermethrin (Pyrethroid), Chlorpyrifos (Organophosphate), Propiconazole (Triazole) and Tebuconazole (Triazole), among others, as well as a combination between two or more AIs. An example of typical formulation using the solvents of this invention is presented in Table 1.

**Table 1: Example of agrochemical formulation in the form of emulsifiable concentrate in alkyl ester of polycarboxylic acids of this invention.**

| COMPONENTS | CONCENTRATION (g/L) |
|---|---|
| Bifenthrin | 200 |
| Admixture of Anionic and non-ionic Surfactant | 100 |
| Alkyl ester policarboxylic | 700 |

The solvency power of this invention can be readily verified by stability measured at low and high temperature, tests being carried out at a temperature of 0°C, 25°C and 54°C, and non-crystallization being proven during the period of 14 days of the active components tested in the typical market concentrations in accordance with Table 2.

**Table 2: Example of typical market concentrations and stability of the solvency power at temperatures of 0.25 and 54°C.**

| ACTIVE INGREDIENT | IN GRAMS PER LITER |
|---|---|
| ABAMECTIN | 18 |
| BIFENTRIN | 200 |
| CYPERMETHRIN | 250 |
| CHLORPYRIFOS | 480 |
| PROPICONAZOLE | 250 |
| TEBUCONAZOLE | 250 |

The solubility limit tests were carried out based on the solubilization of partial portions of 50 at 50 g/L of each active ingredient up to the permanence of the insoluble crystals under agitation at temperature of 25°C. As of this point, a new preparation was re-made in the prior previously soluble concentration under these conditions, portions of 10g/L being added thereafter until attaining the new condition of formation of crystals to return to the prior soluble condition and advance from 1 to 1 g/L thus arriving at the amounts described in Table 3.

**Table 3: Example of solubility limits at 25°C for some classes of active ingredients in alkyl esters of polycarboxylic acids of this invention:**

| **ACTIVE INGREDIENT** | **IN GRAMS PER LITER** |
|---|---|
| **ABAMECTIN** | **116** |
| **BIFENTRIN** | **702** |
| **CYPERMETHRIN** | **532** |
| **CHLORPYRIFOS** | **900** |
| **PROPICONAZOLE** | **1568** |
| **TEBUCONAZOLE** | **240** |

## Claims

1. A polycarboxylic acid ester alkyl derived from branched and straight alcohol of plant origin, **characterized by** comprising C4 and C5 chain alcohols or admixtures thereof adding up to more than 90% by weight of the total esters.

2. The alkyl ester as claimed in claim 1, characterized wherein the alcohols are obtained from plant origin containing between 4 and 5 carbon atoms in both straight and branched form.

3. The alkyl ester as claimed in either of claims 1 or 2, **characterized by** obtaining from polycarboxylic acids preferably citric acid.

4. A use of an alkyl ester in agrochemical formulations, whose alkyl ester is as defined in any of claims 1 to 3, **characterized by** the solubilization of the active ingredient abamectin in the preferred mass concentration by volume of 0.1 to 116 g/L.

5. A use of an alkyl ester in agrochemical formulations, whose alkyl ester is as defined in any of claims 1 to 3 **characterized by** the solubilization of the active ingredient bifenthrin in the preferred mass concentration by volume of 0.1 to 702 g/L.

6. A use of an alkyl ester in agrochemical formulations, whose alkyl ester is as defined in any of claims 1 to 3 **characterized by** the solubilization of the active ingredient cypermethrin in the preferred mass concentration by volume of 0.1 to 532 g/L.

7. A use of an alkyl ester in agrochemical formulations, whose alkyl ester is as defined in any of claims 1 to 3 **characterized by** the solubilization of the active ingredient chlorpyrifos in the preferred mass concentration by volume of 0.1 to 900 g/L.

8. A use of an alkyl ester in agrochemical formulations, whose alkyl ester is as defined in any of claims 1 to 3 **characterized by** the solubilization of the active ingredient propiconazole in the preferred mass concentration by volume of 0.1 to 1568 g/L.

9. A use of an alkyl ester in agrochemical formulations, whose alkyl ester is as defined in any of claims 1 to 3 **characterized by** the solubilization of the active ingredient tebuconazole in the preferred mass concentration by volume of 0.5 to 240 g/L.
